# EUROPEAN PATENT SPECIFICATION

(11) **EP 2 115 169 B1**
(45) Date of publication and mention of the grant of the patent: **02.01.2013**
(21) Application number: 08708666.6
(22) Date of filing: 04.02.2008
(51) Int. Cl.: C12Q 1/68, C12Q 1/70, G01N 33/576

(54) **METHODS FOR DETERMINING DRUG SENSITIVITY IN PATIENTS INFECTED WITH HCV**
VERFAHREN ZUR BESTIMMUNG DER ARZNEIMITTELEMPFINDLICHKEIT HCV-INFIZIERTER PATIENTEN
PROCÉDÉS PERMETTANT DE DÉTERMINER LA SENSIBILITÉ À UN MÉDICAMENT D'UN PATIENT INFECTÉ PAR LE VIRUS DE L'HÉPATITE C

(30) Priority: 05.02.2007 EP 07300773
(43) Date of publication of application: 11.11.2009
(73) Proprietor: INSERM (Institut National de la Santé et de la Recherche Médicale), 75013 Paris (FR); Assistance Publique - Hôpitaux de Paris, 75004 Paris (FR); Université René Descartes (Paris V), 75006 Paris (FR); Université Paris 7 - Denis Diderot, 75005 Paris (FR)
(72) Inventor: VIDAUD, Michel, F-94120 Fontenay-sous-bois (FR); BIECHE, Ivan, F-92150 Suresnes (FR); ASSELAH, Tarik, F-75890 Paris Cedex 18 (FR); MARCELLIN, Patrick, F-75017 Paris (FR)
(74) Representative: Holtz, Béatrice
(86) International application number: PCT/EP2008/051364
(87) International publication number: WO 2008/095907

(56) References cited:
- YEUNG ET AL: "Microarrays and Prediction of Interferon Response in Chronic Hepatitis C: Not Yet Ready for Prime Time" CLINICAL GASTROENTEROLOGY AND HEPATOLOGY, AMERICAN GASTROENTEROLOGICAL ASSOCIATION,, US, vol. 3, no. 12, December 2005 (2005-12), pages 1192-1194, XP005209101 ISSN: 1542-3565
- CHEN ET AL: "Hepatic Gene Expression Discriminates Responders and Nonresponders in Treatment of Chronic Hepatitis C Viral Infection" GASTROENTEROLOGY, ELSEVIER, PHILADELPHIA, PA, US, vol. 128, no. 5, May 2005 (2005-05), pages 1437-1444, XP005314556 ISSN: 0016-5085 cited in the application
- "Affymetrix GeneChip Human Genome U133 Array Set HG-U133A" GEO EXPRESSION, 11 March 2002 (2002-03-11), XP002361324
- BIECHE I ET AL: "Molecular profiling of early stage liver fibrosis in patients with chronic hepatitis C virus infection" VIROLOGY, ACADEMIC PRESS,ORLANDO, US, vol. 332, no. 1, 5 February 2005 (2005-02-05), pages 130-144, XP004715298 ISSN: 0042-6822 cited in the application
- PARADIS ET AL: "Serum Proteome to Predict Virologic Response in Patients With Hepatitis C Treated by Pegylated Interferon Plus Ribavirin" GASTROENTEROLOGY, ELSEVIER, PHILADELPHIA, PA, US, vol. 130, no. 7, June 2006 (2006-06), pages 2189-2197, XP005475320 ISSN: 0016-5085 cited in the application
- HAYASHIDA ET AL: "Pretreatment Prediction of Interferon-Alfa Efficacy in Chronic Hepatitis C Patients" CLINICAL GASTROENTEROLOGY AND HEPATOLOGY, AMERICAN GASTROENTEROLOGICAL ASSOCIATION,, US, vol. 3, no. 12, December 2005 (2005-12), pages 1253-1259, XP005209110 ISSN: 1542-3565 cited in the application
- ASSELAH T ET AL: "Liver gene expression signature to predict response to pegylated interferon plus ribavirin combination therapy in patients with chronic hepatitis C", GUT, BRITISH MEDICAL ASSOCIATION, LONDON, UK, vol. 57, no. 4, 1 April 2008 (2008-04-01), pages 516-523, XP002668777, ISSN: 0017-5749, DOI: 10.1136/GUT.2007.128611 [retrieved on 2007-09-25]

### SEQUENCE LISTING

<110> INSERM (Institut National de la Santé et de la Recherche Médicale)
<120> Methods and kits for determining drug sensitivity in patients infected
   with HCV
<130> U934-EP - INSERM
<160> 9
<170> PatentIn version 3.1
<210> 1
   <211> 656
   <212> DNA
   <213> Homo sapiens
<400> 1
   gggaacacat ccaagcttaa gacggtgagg tcagcttcac attctcagga actctccttc     60
   tttgggtctg gctgaagttg aggatctctt actctctagg ccacggaatt aacccgagca    120
   ggcatggagg cctctgctct cacctcatca gcagtgacca gtgtggccaa agtggtcagg    180
   gtggcctctg gctctgccgt agttttgccc ctggccagga ttgctacagt tgtgattgga    240
   ggagttgtgg ctgtgcccat ggtgctcagt gccatgggct tcactgcggc gggaatcgcc    300
   tcgtcctcca tagcagccaa gatgatgtcc gcggcggcca ttgccaatgg gggtggagtt    360
   gcctcgggca gccttgtggc tactctgcag tcactgggag caactggact ctccggattg    420
   accaagttca tcctgggctc cattgggtct gccattgcgg ctgtcattgc gaggttctac    480
   tagctccctg cccctcgccc tgcagagaag agaaccatgc caggggagaa ggcacccagc    540
   catcctgacc cagcgaggag ccaactatcc caaatatacc tggggtgaaa tataccaaat    600
   tctgcatctc cagaggaaaa taagaaataa agatgaattg ttgcaactct tcaaaa        656
<210> 2
   <211> 2545
   <212> DNA
   <213> Homo sapiens
<400> 2
   atccaataca ggagtgactt ggaactccat tctatcacta tgaagaaaag tggtgttctt     60
   ttcctcttgg gcatcatctt gctggttctg attggagtgc aaggaacccc agtagtgaga    120
   aagggtcgct gttcctgcat cagcaccaac caagggacta tccacctaca atccttgaaa    180
   gaccttaaac aatttgcccc aagcccttcc tgcgagaaaa ttgaaatcat tgctacactg    240
   aagaatggag ttcaaacatg tctaaaccca gattcagcag atgtgaagga actgattaaa    300
   aagtgggaga aacaggtcag ccaaaagaaa aagcaaaaga atgggaaaaa acatcaaaaa    360
   aagaaagttc tgaaagttcg aaaatctcaa cgttctcgtc aaaagaagac tacataagag    420
   accacttcac caataagtat tctgtgttaa aaatgttcta ttttaattat accgctatca 480
   ttccaaagga ggatggcata taatacaaag gcttattaat ttgactagaa aatttaaaac 540
   attactctga aattgtaact aaagttagaa agttgatttt aagaatccaa acgttaagaa 600
   ttgttaaagg ctatgattgt ctttgttctt ctaccaccca ccagttgaat ttcatcatgc 660
   ttaaggccat gattttagca atacccatgt ctacacagat gttcacccaa ccacatccca 720
   ctcacaacag ctgcctggaa gagcagccct aggcttccac gtactgcagc ctccagagag 780
   tatctgaggc acatgtcagc aagtcctaag cctgttagca tgctggtgag ccaagcagtt 840
   tgaaattgag ctggacctca ccaagctgct gtggccatca acctctgtat ttgaatcagc 900
   ctacaggcct cacacacaat gtgtctgaga gattcatgct gattgttatt gggtatcacc 960
   actggagatc accagtgtgt ggctttcaga gcctcctttc tggctttgga agccatgtga 1020
   ttccatcttg cccgctcagg ctgaccactt tatttctttt tgttcccctt tgcttcattc 1080
   aagtcagctc ttctccatcc taccacaatg cagtgccttt cttctctcca gtgcacctgt 1140
   catatgctct gatttatctg agtcaactcc tttctcatct tgtccccaac accccacaga 1200
   agtgctttct tctcccaatt catcctcact cagtccagct tagttcaagt cctgcctctt 1260
   aaataaacct ttttggacac acaaattatc ttaaaactcc tgtttcactt ggttcagtac 1320
   cacatgggtg aacactcaat ggttaactaa ttcttgggtg tttatcctat ctctccaacc 1380
   agattgtcag ctccttgagg gcaagagcca cagtatattt ccctgtttct tccacagtgc 1440
   ctaataatac tgtggaacta ggttttaata attttttaat tgatgttgtt atgggcagga 1500
   tggcaaccag accattgtct cagagcaggt gctggctctt tcctggctac tccatgttgg 1560
   ctagcctctg gtaacctctt acttattatc ttcaggacac tcactacagg gaccagggat 1620
   gatgcaacat ccttgtcttt ttatgacagg atgtttgctc agcttctcca acaataagaa 1680
   gcacgtggta aaacacttgc ggatattctg gactgttttt aaaaaatata cagtttaccg 1740
   aaaatcatat aatcttacaa tgaaaaggac tttatagatc agccagtgac caaccttttc 1800
   ccaaccatac aaaaattcct tttcccgaag gaaaagggct ttctcaataa gcctcagctt 1860
   tctaagatct aacaagatag ccaccgagat ccttatcgaa actcatttta ggcaaatatg 1920
   agttttattg tccgtttact tgtttcagag tttgtattgt gattatcaat taccacacca 1980
   tctcccatga agaaagggaa cggtgaagta ctaagcgcta gaggaagcag ccaagtcggt 2040
   tagtggaagc atgattggtg cccagttagc ctctgcagga tgtggaaacc tccttccagg 2100
   ggaggttcag tgaattgtgt aggagaggtt gtctgtggcc agaatttaaa cctatactca 2160
   ctttcccaaa ttgaatcact gctcacactg ctgatgattt agagtgctgt ccggtggaga 2220
   tcccacccga acgtcttatc taatcatgaa actccctagt tccttcatgt aacttccctg 2280
   aaaaatctaa gtgtttcata aatttgagag tctgtgaccc acttaccttg catctcacag 2340
   gtagacagta tataactaac aaccaaagac tacatattgt cactgacaca cacgttataa 2400
   tcatttatca tatatataca tacatgcata cactctcaaa gcaaataatt tttcacttca 2460
   aaacagtatt gacttgtata ccttgtaatt tgaaatattt tctttgttaa aatagaatgg 2520
   tatcaataaa tagaccatta atcag 2545
<210> 3
   <211> 634
   <212> DNA
   <213> Homo sapiens
<400> 3
   cggctgagag gcagcgaact catctttgcc agtacaggag cttgtgccgt ggcccacagc 60
   ccacagccca cagccatggg ctgggacctg acggtgaaga tgctggcggg caacgaattc 120
   caggtgtccc tgagcagctc catgtcggtg tcagagctga aggcgcagat cacccagaag 180
   attggcgtgc acgccttcca gcagcgtctg gctgtccacc cgagcggtgt ggcgctgcag 240
   gacagggtcc cccttgccag ccagggcctg ggccctggca gcacggtcct gctggtggtg 300
   gacaaatgcg acgaacctct gagcatcctg gtgaggaata acaagggccg cagcagcacc 360
   tacgaggtcc ggctgacgca gaccgtggcc cacctgaagc agcaagtgag cgggctggag 420
   ggtgtgcagg acgacctgtt ctggctgacc ttcgagggga agcccctgga ggaccagctc 480
   ccgctggggg agtacggcct caagcccctg agcaccgtgt tcatgaatct gcgcctgcgg 540
   ggaggcggca cagagcctgg cgggcggagc taagggcctc caccagcatc cgagcaggat 600
   caagggccgg aaataaaggc tgttgtaaga gaat 634
<210> 4
   <211> 23
   <212> DNA
   <213> artificial sequence
<220>
   <223> primer
<400> 4
   tgggtactct gcagtcactg gga 23
<210> 5
   <211> 18
   <212> DNA
   <213> artificial sequence
<220>
   <223> primer
<400> 5
   ccgcaatggc agaccaat 18
<210> 6
   <211> 25
   <212> DNA
   <213> artificial sequence
<220>
   <223> primer
<400> 6
   atccacctac aatccttgaa agacc 25
<210> 7
   <211> 26
   <212> DNA
   <213> artificial sequence
<220>
   <223> primer
<400> 7
   tccattcttc agtgtagcaa tgattt 26
<210> 8
   <211> 24
   <212> DNA
   <213> artificial sequence
<220>
   <223> primer
<400> 8
   gaggcagcga actcatcttt gcca 24
<210> 9
   <211> 21
   <212> DNA
   <213> artificial sequence
<220>
   <223> primer
<400> 9
   ccgccagcat cttcaccgtc a 21
1
1

## Description

### FIELD OF THE INVENTION

The present invention relates to an *in vitro* method for determining whether a patient infected with HCV is a responder or a non-responder to a treatment with a combination of interferon-alpha and ribavirin.

### BACKGROUND OF THE INVENTION

HCV is a single-stranded positive RNA virus, which belongs to the family of *Flaviviridæ,* genus *Hepacivirus.* The genome of HCV comprises a single positive-stranded RNA that encodes a polyprotein of about 3010 amino acid residues, flanked at either end by noncoding regions (NCRs). The 5'-NCR and the first part of the region encoding the polyprotein fold into a complex structure of hairpin loops and unpaired regions that can act as an internal ribosome entry site. This means that translation of the virus genome is cap-independent with the initiation of translation directed to the AUG codon at the beginning of the polyprotein rather than the most 5'-terminal AUG. RNA secondary structures have also been described for the 3'-untranslated region, and it is thought that these might play a role in the replication of the virus genome, although there is at present no direct evidence for this. The N-terminus of the polyprotein is comprised of three structural proteins (core, E1 and E2) and release of these proteins from the polyprotein is dependent upon the signal peptidase associated with the cellular endoplasmic reticulum. Release of the six nonstructural proteins (NS2, NS3, NS4A, NS4B, NS5A and NS5B) from the remainder of the polyprotein is mediated by the virus NS2-NS3 and NS3-NS4A proteases.

Hepatitis C virus infection is among the leading cause of chronic liver diseases with a worldwide prevalence of approximately 300 million people. The severity of disease varies widely from asymptomatic chronic infection to cirrhosis and hepatocellular carcinoma (Seeff et al.2002; Marcellin et al.2002). The progression of fibrosis in chronic hepatitis C determines the ultimate prognosis and thus the need and urgency of therapy (NIH 2002, EASL consensus 2002).

The main treatment goal in patients with chronic HCV infection is the prevention of progressive hepatic fibrosis by eradicating virus. Recently, advances have been made in the treatment of chronic hepatitis C (CHC) with combination therapy with pegylated interferon (PEG-IFN) plus ribavirin (1-ß-D-ribofuranosyl-1,2,4-triazole-3-carboxamide), achieving higher sustained virologic response (SVR) rates than with regimen of standard interferon alpha (IFN) plus ribavirin (Manns et al.2001; Fried et al.2001; Hadziyannis et al.2004). Several cofactors may significantly influence patient response such as genotype and advanced liver fibrosis. As a matter of fact, in patients with genotype 1 infection, the optimal regimen consists in full doses of pegylated interferon (180 ug/week of alpha 2a or 1.5 ug/kg/week of alpha 2b) combined with ribavirin (1000 to 1200 mg/day) for 48 weeks and achieve response rate of 41 to 52% (Manns et al.2001; Fried et al.2001; Hadziyannis et al.2004). In patients with non-1 genotype (particularly genotypes 2 and 3), the optimal response rates (75 to 81%) is achieved with 24 weeks course of full doses of pegylated interferon and reduced doses of ribavirin (800 mg/day).

Despite recent progress in antiviral drug development, the treatment of patients with chronic HCV infection remains a challenge both in term of clinical- and cost-effectiveness.

Furthermore, interferon and ribavirin combination therapy produces a number of well-described deleterious side effects such as fatigue, influenza-like symptoms, hematologic abnormalities, and neuropsychiatric symptoms. Combination therapy with pegylated interferons (peginterferon alfa-2a and alfa-2b) yields an adverse event profile similar to standard interferon, although the frequency of certain adverse events may vary. Premature withdrawal from therapy due to adverse events was required in 10% to 14% of participants in registration trials of these agents (Manns et al.2001; Fried et al.2001; Hadziyannis et al.2004).

Considering the number of side effects and treatment costs, prediction of virologic nonresponse early during therapy or even before starting therapy is potentially useful (NIH 2002, EASL consensus 2002). Therefore, many attempts have been made to find out new methods for predicting whether a patient infected with HCV would show a sustained viral response to a treatment with interferon-alpha and ribavirin.

For instance, document US 2005/0014159 A1 discloses a method for detecting a propensity of an individual to response effectively to treatment of interferon alpha and ribavirin combined therapy which comprises analysing polymorphism profile of CD81 gene.

Recently, Chen el al. (2005) used gene expression profiling (DNA microarrays) and built a classifier comprising 18 genes for predicting the response of an individual to the treatment with pegylated interferon-alpha and ribavirin. However the technology of DNA microarrays is not applicable in routine. Furthermore, this previous study has some limitations. First, they analyzed few patients without validation set. Secondly, the technology of microarray platforms utilised for gene expression analysis needs to be improved for analytical procedures and the use of large numbers of patients for validation.

The purpose of the present invention is to fulfil this need by providing a new reliable method for determining whether a patient is responder to the treatment with interferons-alpha and ribavirin.

### SUMMARY OF THE INVENTION

This invention pertains to *in vitro* methods for determining whether a patient infected with HCV is a responder or a non-responder to a pharmaceutical treatment with a combination of interferon-alpha and ribavirin, the said method comprising a step (a) of determining the expression level of the IFI27 and CXCL9 marker genes in a biological sample previously collected from the said patient.

In some embodiments, step (a) further comprises determining the expression level of the G1 P2 (also known as ISG15) marker gene.

Also disclosed are kits for performing the *in vitro* method above.

These kits encompass DNA microarrays comprising probes that hybridize specifically with the expression products of each of the marker genes tested, including mRNAs and proteins.

### BRIEF DESCRIPTION OF THE FIGURES

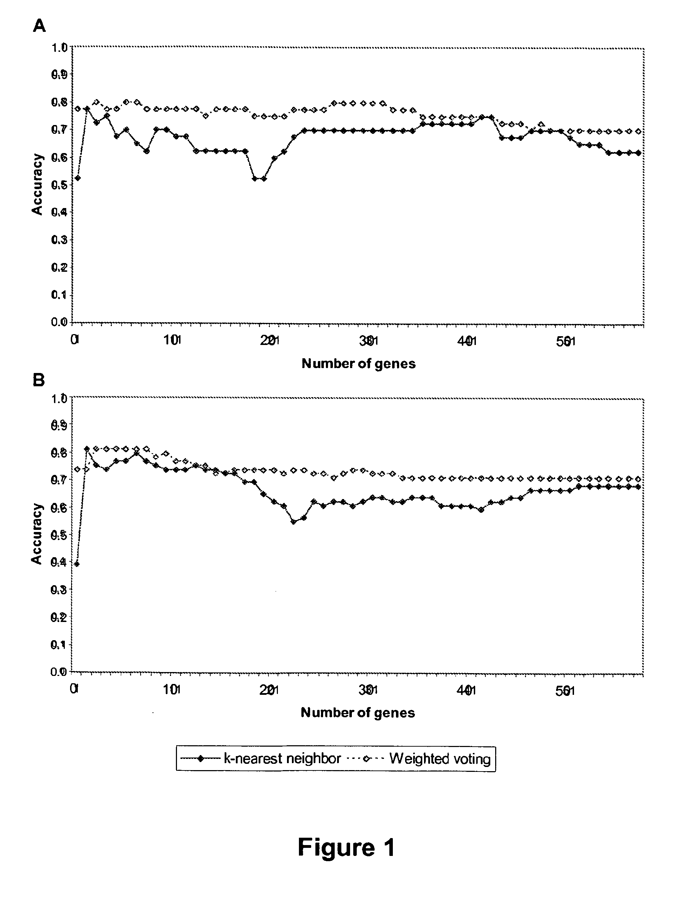

**Figure 1****. :** LOOCV classification accuracies of supervised training models of increasing size in Group A (A) and in the whole dataset (69 patients) (B) using k-nearest neighbor and weighted voting classifiers.

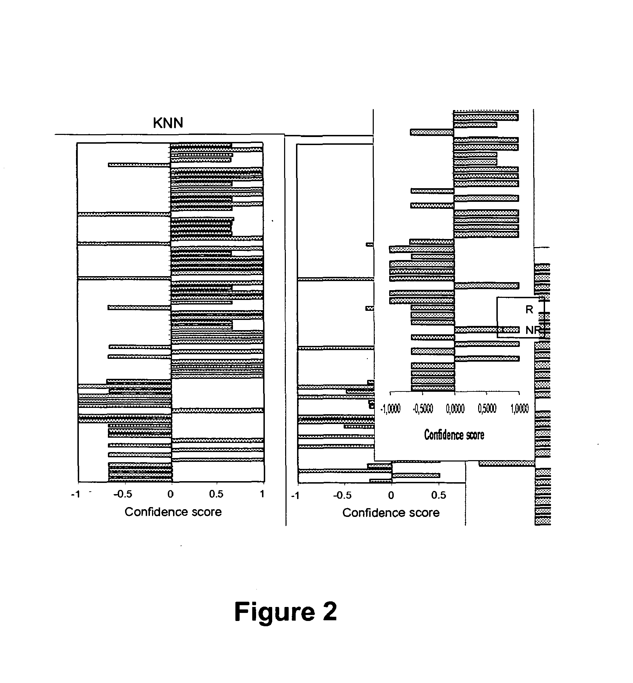

**Figure 2****. :** Results of the KNN and WV classifier analyses on the whole dataset (69 patients) using the two-gene (IFI27, CXCL9) and the three-gene (IFI27, CXCL9, GIP2) predictor sets, respectively. Confidence scores in favour of NR are presented as negative values, and confidence scores of class assignments in favour of SVR are presented as positive values. The actual origins of the patients are indicated by color (SVR, blue; NR: red). The overall accuracy of class assignment using the two-gene predictor with KNN was 81.2% (56/69) in the whole sample, in which 17 of 23 NRs were correctly classified as NRs, and 39 of 46 SVR were correctly classified as SVR. The overall classification accuracy of the three-gene predictor using WV was 81.2% (56/69) in the whole sample, in which 14 of 23 NRs were correctly classified as NR, and 42 of 46 SVR were correctly classified as Rs.

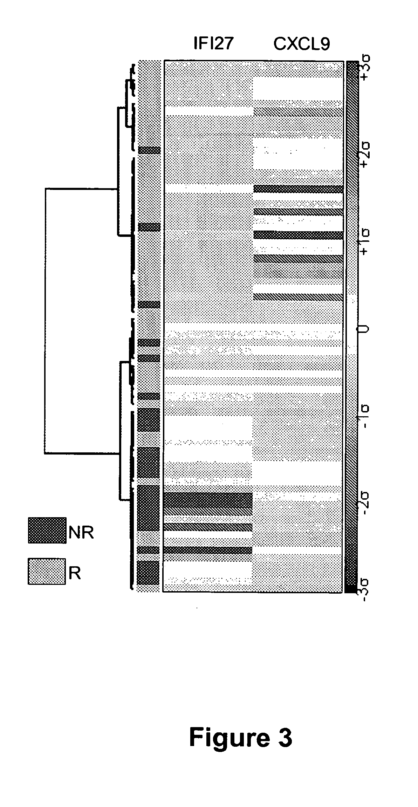

**Figure 3** **:** Unsupervised hierarchical clustering analysis of the 69 patients with chronic HCV using the two-gene signature (IFI27 and CXCL9) identified with the KNN algorithm. A heat map of gene expression patterns of the two genes is presented; relative expression is indicated according to the color scale of blue (low expression) to red (high expression) as indicated in the key.

### DETAILED DESCRIPTION OF THE INVENTION

The inventors have shown that the treatment outcome in patients with HCV chronic infection receiving pegylated interferon plus ribavirin may be accurately predicted by determining the expression level of at least two marker genes, namely the IF127 and the CXCL9 marker genes..

The inventors have selected 58 genes known in the art for being involved in various cellular and molecular mechanisms associated with liver gene expression during chronic hepatitis C in humans (Chen et al.2005; Honda et al.2001; Smith et al.2003; Mihm et al.2004; Bièche et al, 2005; Asselah T, et al. 2005, Helbig et al. 2005, Gale et al. 2005) and have used real-time quantitative RT-PCR assays to compare the mRNA expression of these 58 selected genes in 69 patients infected with HCV that were treated with a combination of pegylated interferon and ribavirin. By using two statistical models, the inventors have identified two specific genes signatures which allow predicting the responsiveness or nonresponsiveness of the HCV-infected patients to the said treatment, respectively : (i) a gene signature comprising two relevant marker genes, namely IF127 and CXCL9 and (ii) a gene signature comprising three relevant marker genes, namely IF127, CXCL9 and G1 P2.

The best classification results were obtained using the three-gene (IF127, CXCL9, and G1P2) signature. The statistical model accurately predicted treatment response for 56 of 69 patients (81.2%).

Thus an object of this invention consists of an *in vitro* method for determining whether a patient infected with HCV is a responder or a non-responder to a pharmaceutical treatment with a combination of interferon-alpha and ribavirin, the said method comprising a step (a) of determining the expression level of the IF127 and CXCL9 marker genes in a biological sample previously collected from the said patient..

### Definitions

The term "hepatitis C virus" or "HCV" is used herein to define a viral species of which pathogenic strains cause hepatitis C, also known as non-A, non-B hepatitis. Based on genetic differences between HCV isolates, the hepatitis C virus species is classified into six genotypes (1-6) with several subtypes within each genotype. Subtypes are further broken down into quasispecies based on their genetic diversity. The preponderance and distribution of HCV genotypes varies globally. For example, in North America, genotype 1 a predominates followed by 1b, 2a, 2b, and 3a. In Europe, genotype 1b is predominant followed by 2a, 2b, 2c, and 3a. Genotypes 4 and 5 are found almost exclusively in Africa. Genotype is clinically important in determining potential response to interferon-based therapy and the required duration of such therapy. Genotypes 1 and 4 are less responsive to interferon-based treatment than are the other genotypes (2, 3, 5 and 6) (Simmonds P et al.; 2005).

A "coding sequence" or a sequence "encoding" an expression product, such as an RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

The term "IFI27 gene" denotes the gene encoding for the interferon alpha-inducible protein 27. IFI27 (also known as ISG12 or P27) belongs to a family of small, interferon-alpha (IFN-)-inducible genes of unknown function. The cDNA for IFI27 was originally cloned as an estrogen-inducible gene in the human epithelial cell line MCF-7 and designated as P27 (Rasmussen et al, 1993). In several types of cell lines (B-lymphoid, cervical and breast epithelial, SV40-transformed keratinocytes, and fibroblasts) IFI27 is strongly induced by IFN-alpha and to a lower extent by IFN-gamma as assessed by RT-PCR (Gjermandsen et al, 2000). Immunohistochemical analyses reveal that upon treatment of HeLa cells with IFN-alpha, the IFI27 gene product resides in the nuclear envelope. IFI27 is thus the first interferon-induced protein that localizes to this site (Martensen et al, 2001). The human polynucleotide sequence is deposited in databases under accession numbers NM_005532 and is shown as SEQ ID NO : 1.

The term "CXCL9 gene" denotes the gene encoding for the chemokine (C-X-C motif) ligand 9. The CXCL9 protein (also known as MIG) is a secreted protein belonging to a family of cytokines believed to be involved in cell-specific chemotaxis, mediation of cell growth and the inflammatory response. Molecules in this family are typically cationic proteins of 70 to 100 amino acids that share four conserved cysteine residues involved in two disulfide bonds. These proteins can be sorted into two groups based on the spacing of the two amino-terminal cysteines. In the first group, the two cysteines are separated by a single residue (C-X-C motif), while in the second group the cysteines are adjacent (C-C motif). The CXCL9 gene product exhibits the C-X-C motif. CXCL9 is a chemoattractant for activated T cells having angiostatic activity. Both CXCL9 and CXCL10 have been considered as important components for the anti-tumor activities of interferon-gamma (IFN-gamma) and interleukin-12 in animal models. Human MIG is induced in THP-1 cells and in peripheral blood mononuclear cells by interferon-gamma but not by interferon-alpha or by lipopolysaccharides. Analysis of mouse and human genomic DNAs suggested that the respective human and murine genes are true mouse-human homologues. The human polynucleotide sequence is deposited in databases under accession numbers NM_002416 and is shown as SEQ ID NO : 2.

The term "G1P2 gene" denotes the gene encoding for the interferon-induced protein 15. G1 P2 (also known as IFI15 and ISG15) is a ubiquitin-like protein that becomes conjugated to many cellular proteins upon activation by interferon-alpha and -beta (Zhao et al., 2005). G1 P2 is a 17 kDa protein encoded by an interferon stimulated gene. While many important functions for ubiquitin have been reported, the functions for G1 P2 and its conjugation are still largely unknown. Evidence suggests that G1 P2 and its modification system play important roles in the innate immune response, regulation of interferon signaling, pregnancy, and several cancers. Modification of proteins by G1 P2 occurs in a manner similar to that of ubiquitin and other ubiquitin-like modifiers. The study of G1P2 and its modification system may yield a set of potentially useful therapeutic targets and thus, there is an increasing awareness and interest in this protein modifier. The human polynucleotide sequences are deposited in databases under accession numbers NM_005101 and is shown as SEQ ID NO : 3.

As used herein, the term "oligonucleotide" refers to a nucleic acid, generally of at least 10, preferably at least 12, more preferably at least 15, and still preferably at least 20 nucleotides, preferably no more than 100 nucleotides, still preferably no more than 70 nucleotides, and which is hybridizable to a genomic DNA, cDNA, or mRNA.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. The terms "primer" and "probe" refer to the function of the oligonucleotide. Typically, contrary to a primer, a probe is not extended by polymerase or ligation following hybridization to the target. A hybridized oligonucleotide may thus function as a probe if it is used to capture or detect a target sequence, and the same oligonucleotide may function as a primer when it is employed as a target binding sequence in an amplification primer.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 11.7-11.8). A minimum length for a hybridizable nucleic acid is at least about 10 nucleotides, preferably at least about 15 nucleotides, and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins modified by, for example, methylation, acetylation, phosphorylation, ubiquitination, SUMOylation, ADP-ribosylation, myristilation, and glycosylation.

The term "sustained viral response" (SVR; also referred to as a "sustained response" or a "durable response"), as used herein, refers to the response of an individual to a treatment regimen for HCV infection, in terms of serum HCV titer. Generally, a "sustained viral response" refers to no detectable HCV RNA (e. g. , less than about 500, less than about 200, or less than about 50 genome copies per milliliter serum) found in the patient's serum for a period of at least about one month, at least about two months, at least about three months, at least about four months, at least about five months, or at least about six months following cessation of treatment.

A "responder" patient, or group of patients, refers to a patient, or group of patients, who show a sustained viral response to the treatment with interferon-alpha and ribavirin. A contrario a "non responder patient" or group of patients, refers to a patient or group of patients, who do not show a sustained viral response to the treatment with interferon-alpha and ribavirin.

The term "interferon-alpha" or "IFN-alpha" as used herein refers to a family of related polypeptides that inhibit viral replication and cellular proliferation and modulate immune response. The term "IFN-alpha" includes naturally occurring IFN-alpha ; synthetic IFN-alpha ; derivatized IFN-alpha (e. g., PEGylated IFN-alpha, glycosylated IFN-alpha, and the like); and analogs of naturally occurring or synthetic IFN-alpha ; essentially any IFN-alpha that has antiviral properties, as described for naturally occurring IFN-alpha. Suitable interferons alpha include, but are not limited to naturally-occurring IFN-alpha (including, but not limited to, naturally occurring IFN-alpha2a, IFN-alpha2b) or recombinant interferon alpha. The term "IFN-alpha" also encompasses derivatives of IFN-alpha that are denvatized (e. g. , are chemically modified) to alter certain properties such as serum half-life. As such, the term "IFN-alpha" includes glycosylated IFN-alpha ; IFN-alpha derivatized with polyethylene glycol ("PEGylated IFN-alpha"); and the like. PEGylated IFN-alpha, and methods for making same, is discussed in, e. g., U. S. Patent Nos. 5,382, 657; 5,981, 709; and 5,951, 974. PEGylated IFN-alpha encompasses conjugates of PEG and any of the above-described IFN-alpha molecules, including, but not limited to, PEG conjugated to interferon alpha-2a (Roferon,Hoffman La-Roche, Nutley, N. J.), interferon alpha 2b (Intron, Schering-Plough, Madison, N. J. ), interferon alpha-2c (Berofor Alpha, Boehringer Ingelheim, Ingelheim, Germany); and consensus interferon as defined by determination of a consensus sequence of naturally occurring interferons alpha (Infergen (InterMune, Inc. , Brisbane,Calif.).

The term "ribavirin" denotes the 1-ß-D-ribofuranosyl-1,2,4-triazole-3-carboxamide compound. Ribavirin is a nucleoside analog available from ICN Pharmaceuticals, Inc. , Costa Mesa,Calif., and is described in the Merck Index, compound No. 8199, Eleventh Edition. Its manufacture and formulation is described in U. S. Pat. No. 4,211, 771.

According to the invention, the term "patient", is intended for a human or non-human mammal infected or likely to be infected with a hepatitis C virus.

### In vitro methods according to the invention:

A first object of the invention consists of an *in vitro* method for determining whether a patient infected with HCV is a responder or a non-responder to a pharmaceutical treatment with a combination of interferon-alpha and ribavirin, the said method comprising a step (a) of determining the expression level of the IFI27 and CXCL9 marker genes in a biological sample previously collected from the said patient.

In a particular embodiment, step (a) of the method further comprises determining the expression level of the G1P2 marker gene.

In a particular embodiment, the method of the invention is suitable for determining whether a patient infected with HCV is responder or non responder to the pharmaceutical treatment with a combination of interferon-alpha and ribavirin, wherein said interferon alpha is pegylated. In preferred embodiments, the pegylated interferon is interferon alpha 2b (Intron, Schering-Plough, Madison, N. J.).

Patients who have been clinically diagnosed as being infected with HCV of any genotype are of particular interest in the invention. However in a preferred embodiment the patient is infected with HCV of genotype 1.

Patients that are infected with HCV are identified as having HCV RNA in their blood, and/or having anti-HCV antibody in their serum. Such individuals include anti-HCV ELISA-positive individuals. Such individuals may also, but need not, have elevated serum alanine aminotransferase (ALT) levels. Individuals who are clinically diagnosed as infected with HCV include naive individuals (e. g. , individuals not previously treated for HCV), particularly those who have not previously received IFN-alpha-based and/or ribavirin-based therapy) and individuals who have failed prior treatment for HCV ("treatment failure" patients).

Biological samples that may be used for performing the *in vitro* method according to the invention encompass any biological sample derived from a patient, including any fluids, tissues, cell samples, organs, biopsies, etc.

In certain embodiments, the biological sample contains nucleic acids. Examples of such samples include fluids, tissues, cell samples, organs, biopsies, etc. Samples may therefore include blood, plasma, saliva, urine, seminal fluid, etc. Peripheral blood is may be used, and mononuclear cells (PBMCs) are the preferred cells. The biological sample may result from a biopsy, and more specifically from a liver biopsy. Generally patient infected with HCV that shall have to be treated with interferon alpha and ribavirin need to have a liver biopsy to find out how extensive their liver damage is before treatment is started. Indeed, the biopsy examination remains the most reliable method to assess the extent of necroinflammatory activity (grade) and fibrosis (stage). The degree of fibrosis has prognostic value and is essential for the indication and the timing of antiviral therapy. Therefore, such biopsy can be useful to perform method of the invention.

In other embodiments, the biological sample contains polypeptides. IFI27, CXCL9, and G1 P2 are secreted proteins, and the inventors thus believe that these proteins also consist of relevant markers for predicting the responsiveness of the HCV-infected patients to the treatment with the combination of interferon-alpha and ribavirin. Therefore, in a particular embodiment, the biological sample is a biological fluid, preferably blood and more preferably serum, wherein the IF127, CXCL9 and G1 P2 proteins are potentially detectable..

An essential feature of the *in vitro* method according to the invention comprises a step (b) of comparing, for each marker gene whose expression level has been determined at step (a), (i) the expression level determined at step (a) with (ii) a control reference value previously determined for the said gene in samples from HCV-infected patients selected from the group consisting of from responder patients , non-responder patients, or both.

Control reference values are easily determinable by the one skilled in the art, by using the same techniques as for determining the expression level of the same marker genes in biological samples previously collected from the patient under testing.

As indicated above, a first control reference value consists of the mean expression level value of the relevant marker genes that has been determined in a group of HCV-infected patients that are not responsive to a treatment with a combination of interferon-alpha and ribavirin.

A second control reference value consists of the mean expression level value of the relevant marker genes that has been determined in a group of HCV-infected patients that are responsive to a treatment with a combination of interferon-alpha and ribavirin.

At the end of step (b) of the *in vitro* method according to the invention, a similarity between (i) the expression level value found for the marker genes in the patient tested and (ii) the first control reference value above indicates that the patient tested consists of a non-responder to the treatment with a combination of interferon-alpha and ribavirin.

At the end of step (b) of the *in vitro* method according to the invention, a similarity between (i) the expression level value found for the marker genes in the patient tested and (ii) the second control reference value above indicates that the patient tested consists of a responder to the treatment with a combination of interferon-alpha and ribavirin.

### Determination of the expression level of the marker genes by quantifying mRNAs

Total RNAs or proteins can be easily extracted therefrom. The biological sample may be treated prior to its use, e.g. in order to render nucleic acids or proteins available. Techniques of cell or protein lysis, concentration or dilution of nucleic acids, are known by the skilled person.

Determination of the expression level of a gene can be performed by a variety of techniques. Generally, the expression level as determined is a relative expression level.

More preferably, the determination comprises contacting the sample with selective reagents such as probes, primers or ligands, and thereby detecting the presence, or measuring the amount, of polypeptides or nucleic acids of interest originally in the sample. Contacting may be performed in any suitable device, such as a plate, microtiter dish, test tube, well, glass, column... In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a particular embodiment, the expression level may be determined by determining the quantity of mRNA. Such method may be suitable to determine the expression levels of IFI27, CXCL9, or G1 P2 genes in a nucleic sample like liver biopsy or PBMCs isolated from blood.

Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then detected by hybridization (e. g., Northern blot analysis).

Alternatively, the extracted mRNA may be subjected to coupled reverse transcription and amplification, such as reverse transcription and amplification by polymerase chain reaction (RT-PCR), using specific oligonucleotide primers that enable amplification of a region in the IFI27, CXCL9, or G1P2 genes. Preferably quantitative or semi-quantitative RT-PCR is preferred. Real-time quantitative or semi-quantitative RT-PCR is particularly advantageous. Extracted mRNA may be reverse-transcribed and amplified, after which amplified sequences may be detected by hybridization with a suitable probe or by direct sequencing, or any other appropriate method known in the art.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

In a particular embodiment, the method of the invention the steps of providing total RNAs obtained from the biological sample of the patient, and subjecting the RNAs to amplification and hybridization to specific probes, more particularly by means of a quantitative or semi-quantitative RT-PCR.

Total RNAs can be easily extracted from a biological sample. For instance, the biological sample may be treated prior to its use, e.g. in order to render nucleic acids available. Techniques of cell or protein lysis, concentration or dilution of nucleic acids, are known by the skilled person.

In another embodiment, the expression level may be determined by DNA microarray analysis. Such DNA microarray or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210)

In this context, the invention further provides a DNA microarray comprising a solid support onto which nucleic acids that are specific for IFI27, CXCL9 gene nucleic acid expression products (i.e. mRNA or cDNA) are immobilized. In specific embodiments, such a DNA microarray may further comprise nucleic acid(s) specific for G1 P2 gene nucleic acid expression product.

### Determination of the expression level of the marker genes by quantifying proteins

Other methods for determining the expression level of the said marker genes, at step (a) of the *in vitro method according to the invention,* include the determination of the quantity of polypeptides encoded by the said genes that is found in the biological sample previously collected from the HCV-infected patient tested. In particular, such methods may be particularly suitable to determine the quantity of IFI27, CXCL9 or G1P2 proteins in a biological sample like serum, since these proteins are secreted.

Such methods comprise contacting a biological sample with a binding partner capable of selectively interacting with a marker protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs: biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of antibodies against the proteins to be tested. A biological sample containing or suspected of containing the marker protein is then added to the coated wells. After a period of incubation sufficient to allow the formation of antibody-antigen complexes, the plate (s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

The method of the invention may comprise a further step consisting of comparing the combined expression level of said genes with control reference values obtained from responder and non-responder groups of patients.

In particular embodiment, the preferred responder group of patient that provides for the reference values is a group that show a sustained viral response which is defined as undetectable HCV RNA determined six months after completion of treatment (NIH Hepatology 2002, EASL Consensus).

### Diagnostic kits :

A further disclosure consists of kits for determining whether a patient infected with HCV is a responder or a non-responder to a treatment with a combination of interferon-alpha and ribavirin, wherein the said kits comprise means for measuring the expression level of IFI27, CXCL9. A kit for determining whether a patient is a non-responder may further comprise means for measuring the expression level of G1 P2.

The said kit may include the following components :
(i) a series of probes directed against the IFI27, CXCL9 (and G1 P2) genes, usually made of DNA, and that may be pre-labelled.; and
(ii) hybridization reagents

Such probes are "sequence-specific oligonucleotides", i.e. oligonucleotides or nucleic acids that are capable of specifically hybridizing with a portion of the IFI27, CXCL9 or G1 P2 gene sequences under conditions of high stringency (Sambrook et al, 1989). Oligonucleotides that may be used as probes can be labelled according to any technique known in the art, such as with radiolabels, fluorescent labels, enzymatic labels, sequence tags, etc. A labelled oligonucleotide may be used as a probe to detect the presence of said genes.

Generally, oligonucleotides are prepared synthetically, preferably on a nucleic acid synthesizer. Accordingly, oligonucleotides can be prepared with non-naturally occurring phosphoester analog bonds, such as thioester bonds, etc.

The kit may also contain other suitably packaged reagents and materials needed for the particular hybridization protocol, including solid-phase matrices, if applicable, and standards.

Alternatively, the probe may be unlabelled and the ingredients for labelling may be included in the kit in separate containers.

The kit may include :
(i) amplification primers specific for the amplification of IFI27, CXCL9 and G1 P2 genes: and
(ii) sequence determination or amplification reagents.

The kit may also contain other suitably packaged reagents and materials needed for the particular sequencing amplification protocol. The kit may comprise a panel of sequencing or amplification primers, whose sequences correspond to sequences that allow the amplification of region in the IFI27, CXCL9 or G1 P2 genes.

Sequencing primers may be pre-labelled or may contain an affinity purification or attachment moiety.

A kit may comprise a series of pair of oligonucleotide primers specific for amplifying all or part of the IFI27, CXCL9 or G1 P2 genes. Preferably said kit may comprise a series of primer pair consisting of oligonucleotides
- SEQ ID NO.4 and SEQ ID NO.5 for the amplification of IFI27 gene
- SEQ ID NO.6 and SEQ ID NO.7 for the amplification of CXCL9 gene
- SEQ ID NO.8 and SEQ ID NO.9 for the amplification of G1 P2 gene

The kit may comprise a series of antibodies that specifically recognized the IFI27, CXCL9 and G1 P2 proteins.

### Therapeutic methods:

Further disclosed is a method for the treatment of an HCV infection or a disease associated with an HCV infection, such as chronic hepatitis C or for the prevention of liver diseases, such as liver fibrosis, liver cirrhosis or hepatocellular carcinoma.

In the context of the invention, the term "treating" or "treatment", as used herein, means reversing, alleviating, inhibiting the progress of, or preventing the disorder or condition to which such term applies, or one or more symptoms of such disorder or condition.

Said method may comprise the following steps:
a) determining whether a patient infected with HCV is a responder or a non responder to a treatment with a combination of interferon-alpha and ribavirin, by performing the *in vitro* method that is described above; and
b) administering a combination of interferon-alpha and ribavirin to the said patient, if the said patient has been determined as consisting of a responder, at step a) above.

The classification of the patient -as responder or non-responder-is made according to the combined expression level of said two or three genes. It allows to define a subgroup of patients who will be responsive or not to a treatment with interferon-alpha and ribavirin.

Said interferon-alpha may be pegylated. Preferably the pegylated interferon is interferon alpha 2b (Intron, Schering-Plough, Madison, N. J.).

### EXAMPLE:

### A. MATERIAL AND METHODS :

### A.1. Patients :

**Chronic Hepatitis C patients :** Percutaneous liver biopsy specimens were selected from a cohort of untreated adult patients with CHC followed at Beaujon Hospital (Clichy, France). In each case, both immediately frozen liver tissue (stored at -80°C) and fixed-paraffin-embedded tissue (for histology) were available. Patients were included in this study if they met the following criteria:
a- An established diagnosis of CHC with detectable anti-HCV antibodies, detectable serum HCV RNA with RT-PCR (HCV Amplicor 2.0; Roche diagnostics, Mannheim, Germany) and findings consistent with chronic hepatitis C on liver biopsy.
b- Absence of other causes of chronic liver disease (undetectable HBs-Ag, no excessive alcohol consumption defined as intake > 30g/day, hemochromatosis, autoimmune hepatitis, Wilson's disease, alpha-1 antitrypsin deficiency, primary sclerosing cholangitis or primary biliary cirrhosis).
c - Standard treatment regimen: all were naïve patients and received the same complete treatment schedule consisting of pegylated-interferon (PEG-IFN) alpha-2b (Viraferon-peg, Schering corp) at a dose of 1.5 µg/kg/week in combination with ribavirin at a dose adjusted according to body weight (<65 kg: 800 mg/day; 65 kg to 85 kg: 1000 mg/day; >85 kg: 1200 mg/day). Duration of treatment was 24 weeks for patients with HCV genotypes 2 or 3 infection, and 48 weeks for patients with HCV genotypes 1 or 4 infection.
d - Adequate follow-up: patients were assessed as outpatients. Detection of HCV RNA in serum was performed at week 12, at the end, and six months after the end of treatment by RT-PCR.

Assessment of efficacy: Sustained virological response (SVR) was defined as undetectable HCV RNA determined six months after completion of treatment (1-2). Non response (NR) was defined as detectable serum HCV RNA at the end of treatment.

We first built a gene signature on a training set with 40 consecutive patients with CHC including 14 non responders and 26 responders (Group A). We then validated the gene signature on an independent validation set of 29 consecutive patients with CHC including 9 non responders and 20 responders (Group B), to confirm that it would generalize to new data. Baseline characteristics of groups A & B are shown in tables 1 & 2.

**Histological normal controls :** Percutaneous liver biopsy specimens, obtained from 8 adults with mildly elevated serum alanine aminotransferase activity sent to Beaujon Hospital (Clichy, France) with no other causes of liver disease (medication, alcohol, chronic viral hepatitis, autoimmune processes, and metabolic disease). In these adults, liver biopsies were performed percutaneously after a local anaesthesia. All these 8 liver tissue specimens were histologically normal (i.e. absence of inflammation, fibrosis and pathological pattern).

### A.2. Real-time RT-PCR :

In prior studies using the same technological approach, we have shown that several altered molecular pathways are involved in hepatitis C compared to the normal liver (Bièche et al. 2005) and in the progression of liver fibrosis (Asselah T, et al. 2005).

**Theoretical basis :** The theoretical and practical aspects of real-time quantitative RT-PCR using the ABI Prism 9700 Sequence Detection System (Perkin-Elmer Applied Biosystems, Foster City, CA), has been described in detail elsewhere (Asselah et al.2005).

The precise amount of total RNA added to each reaction mix (based on optical density) and its quality (i.e. lack of extensive degradation) are both difficult to assess. We therefore also quantified transcripts of two endogenous RNA control genes involved in two cellular metabolic pathways, namely TBP (Genbank accession NM_003194), which encodes the TATA box-binding protein (a component of the DNA-binding protein complex TFIID), and RPLP0 (also known as 36B4; NM_001002), which encodes human acidic ribosomal phosphoprotein P0. Each sample was normalized on the basis of its TBP (or RPLPO) content.

Results, expressed as N-fold differences in target gene expression relative to the TBP (or RPLPO) gene, and termed "Ntarget", were determined as Ntarget = 2^{ΔCtsample} where the ΔCt value of the sample was determined by subtracting the average Ct value of the target gene from the average Ct value of the TBP (or RPLPO) gene.

The Ntarget values of the samples were subsequently normalized such that the median of the normal histologic liver Ntarget values was 1.

**Selection of genes :** Before treatment we suspected that liver gene expression could differentiate responders from non responders. By studying the literature, we selected 58 genes involved in various cellular and molecular mechanisms associated with liver gene expression during chronic hepatitis C in humans (Chen et al.2005; Honda et al.2001; Smith et al.2003; Mihm et al.2004; Bièche et al, 2005; Asselah T, et al. 2005, Helbig et al. 2005, Gale et al. 2005...). We especially focused on genes whose expression was dysregulated during HCV infection compared to the normal histological liver (n = 37; Bièche et al.2005), genes involved in the progression of fibrosis in CHC (n = 7, Asselah et al. 2005), those associated with treatment outcome (n = 18, Chen et al. 2005), and genes associated with immune response to HCV (Helbig et al. 2005). These genes mainly encode proteins involved in the immune response, extracellular remodelling, and interferon signalling (see Table 3).

**Primers and controls :** Primers for TBP, RPLPO and the 58 target genes were chosen with the assistance of the Oligo 5.0 computer program (National Biosciences, Plymouth, MN).

We conducted searches in the dbEST and nr databases to confirm the total gene specificity of the nucleotide sequences chosen as primers, and the absence of single nucleotide polymorphisms. In particular, the primer pairs were selected to be unique in relation to the sequences of closely related family member genes or of the corresponding retropseudogenes. To avoid amplification of contaminating genomic DNA, one of the two primers was placed at the junction between two exons, if possible. In general, amplicons were between 70 and 120 nucleotides long. Gel electrophoresis was used to verify the specificity of PCR amplicons.

For each primer pair, no-template control (NTC) and no-reverse-transcriptase control (RT negative) assays were performed and produced negligible signals (usually > 40 in Ct value), suggesting that primer-dimer formation and genomic DNA contamination effects were negligible. The RNA extraction, cDNA synthesis and PCR reaction conditions are described elsewhere (Asselah et al. 2005).

Specific primer for IFI27, CXCL9 and G1 P2 were described in Table 4 :

### A.4. Statistical analysis :

**Significance testing, clustering and supervised class prediction :** Genes differentially expressed between SVR and NR were identified using the signal-to-noise and Student's t-test statistics. The signal-to-noise ratio (SNR) measures the difference in means (the signal) in each of the two classes, i.e., the SVR and NR groups, scaled by the sum of the standard deviations (the noise): SNR= (µ0 - µ1)/(σ0 + σ1) where µi. σi are the mean and standard deviation of class i. Compared to the t statistic, defined as (µ0 - µ1)/( σ02 + σ12)1/2, which assumes equal variance across each of the groups, the SNR statistic penalizes genes that have a higher variance in each class more than those that have a high variance in one class and a low variance in another. P-values were estimated using 10,000 permutations of the sample status (SVR/NR) and genes with an adjusted P-value less than 0.05 were considered to be significantly differentially expressed genes. P-values were corrected using the Benjamini and Hochberg false discovery rate (FDR) method (Benjammi and Hochberg, 1995) to control false positive results from multiple testing.

Unsupervised hierarchical clustering of expression data was performed using average linkage clustering with cosine distance as similarity metric. Cluster analysis is a technique to automatically group and find structures in a dataset. It organizes the data into a tree structure based on the degree of similarity between gene expression profiles; the highest levels of the tree represent the discovered classes.

Supervised class prediction analysis was performed using k nearest neighbor (KNN) and weighted voting (WV) prediction algorithms to achieve optimal prediction accuracy for sample classification with a minimal set of predictors. Briefly, a KNN classifier (Cover and Hart, 1967) is based on a distance function between pairs of observations and identifies the class of a testing sample by determining its k nearest neighbors in the training dataset and by choosing the most common class of those k neighbors. Our KNN classifier used cosine distance and a distance-weighted voting scheme. The number of k neighbors was chosen by cross-validation (CV) on the training set, and the k yielding the smallest CV error rate was retained for use on the test set. The WV classifier makes a weighted linear combination of relevant marker genes obtained in the training set to provide a classification scheme for new samples. The selection of classifier input features was performed by computing the SNR statistic.

Prediction models were built using gene expression data from Group A samples, which served as a training set. Models containing increasing numbers of genes were evaluated by leave-one-out cross validation (LOOCV) to identify the smallest predictive model yielding the most accurate class assignments. In LOOCV, one sample from the training set is excluded, and the rest of the training samples are used to build the classifier. Then the classifier is used to predict the class of the left out one, and this is repeated for each sample in the training set. The LOOCV estimate of classification accuracy is the overall number of correctly classified samples, divided by the number of samples in the training set. Thereafter, a validation analysis of the predictive classification model was performed using an independent test set (Group B), and the overall accuracy of class assignment for samples in the test set was reported.

All analyses were performed using GenePattern version 2.0 (http://www.broad.mit.edu/cancer/software/genepattern) (Reich et al, 2006).

### B. RESULTS :

**B.1. Identifying significantly differentially expressed genes among SVR and NR :** We first examined whether there were significant differences in gene expression between SVR and NR liver tissue biopsy specimens. Tables 5 and 6 show the results of the Student's t tests when applied to Groups A and B, respectively; similar results were obtained with the SNR statistic pointing to the same differentially expressed genes, although not ranked exactly in the same order.

Genes correlated with SVR/NR statuses were identified by sorting all of the genes according to the t-test statistic. Table 3 shows for group A, the 10 genes with a non-adjusted P-value of less than 0.05. Although this does not strictly assess significance, these P-values are useful to identify genes that should be studied further. From the Group A data, we identified three genes whose expression differed significantly between SVR and NR (FDR<0.05): G1 P3 (also known IFI6), IFI27 and G1P2 (Table 5). Note that G1P2 and G1P3 were already identified as differentially expressed genes between SVR and NR (with P < 0.001) in a study performed on an independent data set (Chen et al., 2005).

When gene expression data from Group B were analysed, 19 genes were found to be differentially expressed (FDR < 0.05) (Table 6). The three genes shown to be significant (FDR < 0.05) in Group A (G1 P3, IFI27 and G1P2) also showed significant differences in their expression profiles between SVR and NR in this independent sample, being among the 4 genes with the highest level of significance. Furthermore, it is interesting to note that among the 10 genes displaying a non-adjusted P-value below 0.05 in Group A, six were differentially expressed (FDR < 0.05) in Group B (IFI27, G1 P2, G1 P3, OAS2, herc5, IFIT1). Among these six genes, five (IFI27, G1P2, G1P3, OAS2, IFIT1) were also identified as differentially expressed by Chen et al. (2005).

**B.2. Selection of the most useful gene subsets for gene expression-based classification :** Exploratory data analysis using unsupervised hierarchical clustering was performed on both Group A and the entire dataset (Group A and Group B) using the whole set of genes (58 genes). No distinctive clusters were identified between the SVR and NR groups by this analysis. This was expected since most of the genes considered were not correlated with NR/SVR status. Hence, the SVR and NR patients could not be differentiated by unsupervised data analysis.

Prediction models were built using two supervised learning classifiers, KNN and WV, for gene signature discovery. Since good predictors do not necessarily have low type 1 errors when the null hypothesis of no association with the clinical outcome is tested for each of the investigated variables (Devlin et al, 2003), the analysis was not restricted to the statistically significant discriminatory genes but was performed using the entire set of genes. The KNN algorithm identified a two-gene classifier (IFI27 and CXCL9, using k=3) that gave the highest overall accuracy (77.5%) in distinguishing between SVR and NR gene expression profiles as evaluated by LOOCV of Group A, used as a training set (Figure 1). Increasing the size of the classifier set did not increase accuracy. This two-gene signature correctly classified 23 of 26 SVR patients (88.5%) and 8 of 14 NR patients (57.1%) (Table 7).

It was validated on an independent test set (Group B) consisting of 29 patients, 23 of whom were correctly classified (79.3%): treatment response was correctly predicted for all NR patients (100%) and 14 of 20 SVR patients (70%).

With the WV classifier, the highest classification accuracy (80.0%) was obtained using a three-gene subset (IFI27, CXCL9, G1P3), and results did not improve if additional genes were included (Table 7). This predictive gene signature also showed good predictive accuracy in the independent test set, with 72.4% (21/29) of samples correctly assigned.

Since loss of classification power could occur if only Group A samples were used in the construction of classification models, we built predictive models using the entire dataset (69 samples) and applied a LOOCV procedure to this sample to estimate the prediction accuracy of the identified gene signatures (Figure 2). The best results were obtained using a two-gene (IF127 and CXCL9, with k=3) and a three-gene (IFI27, CXCL9, and G1P2) subset when performing KNN and WV analyses, respectively. Both predictive models accurately predicted treatment response for 56 of 69 patients (81.2%) with chronic HCV (Table 6). However, while the prediction errors within the NR and SVR groups of patients were similar with the two-gene signature KNN classifier (26.1% and 15.2%, respectively), they were markedly unbalanced when training the WV algorithm with the three-gene subset (39.1% and 8.7%, respectively) (Figure 2). Interestingly, among the genes selected as good predictors for gene expression-based classification of treatment outcomes, CXCL9 was not shown to be differentially expressed between SVR and NR patients (FDR > 0.05). When we performed unsupervised clustering analysis on the whole dataset (69 samples) using the two-gene signature (IFI27, CXCL9), the resulting dendogram aggregated SVR patients into one cluster and the other cluster was further subdivided into two subclusters: one containing SVR and the other NR patients (Figure 3). Six NR and seven SVR patients were incorrectly classified in this tree, which is consistent with the results obtained with the KNN classifier. Moreover, when we tested the two-gene signature in the group of patients with genotype 1 infection, 31 of 41 patients (75.6%) were correctly classified using KNN, with a predictive accuracy of 71.4% (15/21) and 80% (16/20) in NR and SVR patients, respectively.

### B.3. DISCUSSION OF THE RESULTS

In this study, we used large scale real time PCR to predict treatment outcome in patients with chronic HCV infection receiving pegylated interferon plus ribavirin. We examined the expression level of 58 genes known to be involved in various cellular and molecular mechanisms associated with response to treatment. All patients were well defined and phenotyped: all were naïve patients and received the same complete treatment schedule of pegylated-interferon (PEG-IFN) alpha-2b (Viraferon-peg, Schering corp) with ribavirin.

Real-time quantitative RT-PCR is a promising alternative to cDNA microarrays for gene profiling. In particular, real-time RT-PCR is far more precise, reproducible and quantitative than cDNA microarrays. Using this approach, we have shown that several altered molecular pathways are involved in the progression of fibrosis in chronic hepatitis C (Asselah T, et al. 2005). Real-time RT-PCR is also more useful for analyzing weakly expressed genes such as cytokines in the present study.

The best classification results were obtained using a two-gene (IFI27 and CXCL9) and a three-gene (IFI27, CXCL9, G1P2) subset (including the two previous genes) when performing KNN and WV analyses, respectively, on the whole data set (Figure 1). Both predictive models accurately predicted treatment response for 56 of 69 patients (81.2%) with chronic HCV.

In patients with HCV genotype 1 infection, virological response rates are less than 50% (Manns et al. 2001, Fried et al. 2002, Hadziyannis et al. 2004). Since a significant number of patients will fail to respond to optimal antiviral treatment, with significant side effects, it is of major interest both economically and for patient care, to predict patients who will fail to respond as early as possible, ideally at baseline (before treatment). For this purpose, the best signature to predict non response is the three gene (IFI27, CXCL9, G1P2) subset. These three genes belong to the interferon stimulating genes family.

Many of the genes founded differentially expressed between SVR and NR in the training set (G1P3, IFI27, G1P2, MIX1, OAS2, IL8, IFIT1) and in the validation set (IFI27, G1P2, G1P3, STAT1, LGS3B, OAS2, FGF7, ISF3G, IFTM1, IFIT1, OAS3, IGP1, PLCR1) belong to IFN-inducible genes. Interestingly, all these genes were up-regulated in SVR and NR samples in comparison with normal liver. It is suggested that IFN-inducible genes are expressed during chronic HCV infection via direct activation of IRF3, NFKB1, or JUN through IFN-dependent signalling events in host cells (Gale and Foy, 2005). Interestingly, the three genes signature (IFI27, CXCL9, G1P2) were founded strongly up-regulated in chronic hepatitis (with mild disease) in comparison with normal liver (Bièche et al.2005). IFI27 and G1P2 are IFN α/β inducible genes with high nucleotide sequence homologies and unknown function (Li et al.2001). CXCL9 is an IFN-γ inducible gene.

In a recent study, expression profiling was performed on percutaneous needle liver biopsy specimens taken before therapy (Chen et al. 2005). Gene expression levels were compared among 15 nonresponders, 16 responders, and 20 normal liver biopsy specimens. Up-regulation of a specific set of IFN-responsive genes predicts nonresponse to treatment with PEG IFN alpha plus ribavirin. Interestingly, five of these genes (IFI27, G1 P2, G1 P3, OAS2, IFIT1) were also identified in our study as being differentially expressed between SVR and NR.

In another study by Hayashida et al.; liver tissue samples prior to treatment by IFN or IFN/ribavirin were analysed by microarray (Hayashida et al.2005). In the IFN group, differentially expressed genes were mainly IFN, lipid metabolism, complement and oxidoreductase-related genes. For IFN/ribavirin combination treatment a different set of genes was identified with cyclophilin A and multidrug resistance protein. SR/TR and NR patients for interferon therapy were successfully predicted using only host data from the complementary DNA microarray. Surprisingly the discriminatory family genes that were identified were different in the 2 groups receiving IFN or IFN/ribavirin.

One study analysing gene expression patterns in peripheral blood mononuclear cells during IFN therapy confirmed up-regulation of genes thought to be IFN-stimulated genes or involved in antigen processing and presentation (Ji X, et al. 2003). However, studies assessing gene expression in peripheral blood mononucelar cells in chronic hepatitis C before treatment are needed.

These three studies have some limitations. First, only a few patients were analyzed without a validation set. Second, patients were heterogenous for treatment received (IFN monotherapy, Pegylated interferon, ribavirin...) and the definition of virological non responders in relation to compliance. Moreover, the diversity of microarray platforms used for gene expression analysis and the variability of microarray data emphasises the need for quality assurance. High quality RNA samples are essential for microarray analysis, and the quality of each RNA preparation must be rigorously assessed. Careful measures must be taken throughout RNA extraction to prevent the RNA from degrading. Furthermore, improved analytical procedures and the use of large numbers of patients are needed for validation. Gene signatures will probably be used in the future for optimised and tailored treatment.

Many of the genes found to be up-regulated between NR and SVR code molecules secreted in the serum (cytokines, interferon pathway, IFI27, CXCL9, G1P2) and provide a logical functional approach for the development of serum markers to predict response to treatment. For instance one study examined the levels of chemokines that bind to CXC chemokine receptor 3 (CXCR3) to determine whether these chemokines might play a role in the failure of the immune system to clear HCV infection (Butera et al. 2005). Levels of CXCL10 and CXCL9 decreased following successful antiviral therapy; CXCL11 did not decline significantly during or in the first 6 months after therapy. The baseline level of CXCL10 (measured before antiviral treatment) was highest in patients with HCV who did not respond to therapy. These results suggest that plasma concentrations of immunoreactive CXCL10 may predict responsiveness or nonresponsiveness to antiviral therapy with pegylated interferon (IFN) with or without ribavirin.

In conclusion, our study has demonstrated that NR and SVR have prior to treatment, different gene expression patterns. The most notable changes in gene expression mainly occurred in the interferon stimulating genes. We used two independent groups of patients (training set and validation set) and we could predict treatment response with a three-gene signature (IFI27, CXCL9, and G1P2). These genes code molecules secreted in the serum and provide a logical functional approach for the development of serum markers to predict response to treatment.

**Table 1 : Patients Training set (group A) characteristics**

| **variable** | **Patients** | **NR** | **SVR** |
|---|---|---|---|
| **No.** | 40 | 14 | 26 |
| **Gender (male, n (%))** | 26 (65) | 9(64) | 17(65) |
| **Age (years)** | 47.4 ± 8.6 | 46.5± 7.1 | 47.9± 9.4 |

| **Source of infection (n(%))** | | | |
|---|---|---|---|
| blood transfusion intravenous drug use unknow | 11 (27.5) | 3 (21.4) | 8 (30.8) |
| | 13 (32.5) | 5 (35.7) | 8 (30.8) |
| | 16 (40.0) | 6 (42.9) | 10 (38.6) |
| **ALT (IU/L, median)** | 120.7 ± 57.1 | 132.0 ± 86.7 | 114.6 ± 43.6 |

| **HCV genotype (n)** | | | |
|---|---|---|---|
| 1 | 24 | 13 | 11 |
| 2 | 3 | 0 | 3 |
| 3 | 8 | 0 | 8 |
| 4 | 4 | 1 | 3 |
| 5 | 1 | 0 | 1 |

| **Stage (n)** | | | |
|---|---|---|---|
| F1 | 10 | 0 | 10 |
| F2 | 17 | 7 | 10 |
| F3 | 8 | 4 | 4 |
| F4 | 5 | 3 | 2 |

**Table 2 : Patients Test set (group B) characteristics**

| **variable** | **Patients** | **NR** | **R** |
|---|---|---|---|
| **No.** | 29 | 9 | 20 |
| **Gender (male, n (%))** | 22 (76) | 8 (89) | 14 (70) |
| **Age (years)** | 45.1 ± 9.1 | 48.2 ± 10.6 | 43.6 ± 8.3 |

| **Source of infection (n(%))** | | | |
|---|---|---|---|
| blood transfusion Intravenous drug use unknown | 3 (10.3) | 1 (11) | 2 (10) |
| | 9 (31.1) | 3 (33) | 6 (30) |
| | 17 (58.6) | 5 (66) | 12 (60) |
| **ALT (IU/L, median)** | 128.58 ± 70.1 | 120.13 ± 48.6 | 132.33 ± 78.7 |

| **HCV genotype (n)** | | | |
|---|---|---|---|
| 1 | 17 | 8 | 9 |
| 2 | 2 | 0 | 2 |
| 3 | 6 | 0 | 6 |
| 4 | 4 | 1 | 3 |
| 5 | 0 | 0 | 0 |

| **Stage (n)** | | | |
|---|---|---|---|
| F1 | 7 | 1 | 6 |
| F2 | 13 | 4 | 9 |
| F3 | 7 | 2 | 5 |
| F4 | 2 | 2 | 0 |

**Table 3 : List of 58 genes**

| Interferon signaling pathway: | | Growth factors : | | Transcription factors : | |
|---|---|---|---|---|---|
| IRF7 | interferon regulatory factor 7 | CCL8 | Chemokine (C-C motif) ligand 2 (MCP-2) | ATF5 | Activating transcription factor 5 |
| ISGF3G | interferon-slimulaled transcription factor 3, gamma 48KD | CXCL10 | Chemokine (C-X-C motif) ligand 10 (IP-10) | | |
| STAT1 | signal transducer and activator of transcription 1 | CXCL11 | Chemokine (C-X-C motif) ligand 11 (IP-9) | Cytoskeletal : | |
| STAT2 | signal transducer and activator of transcription 2 | CXCL6 | Chemokine (C-X-C motif) ligand 6 (GCP-2) | | |
| | | CXCL9 | Chemokine (C-X-C motif) ligand 9 (MIG) | STMN2 | Stathmin-like 2 (SCG 10) |
| | | FGF7 | Fibroblast growth factor 7 (KGF) | | |
| | | IFNG | Interferon, gamma | Iron metabolism: | |
| Interferon-inducible proteins : | | IL6 | Interleukin 6 | | |
| | | IL8 | Interleukin 8 | HAMP | Hepeidin antimicrobial peptide |
| G1P2 | interferon, alpha-inducible protein (clone IFI-15K) | IL10 | Interleukin 10 | | |
| G1P3 | interferon, alpha-inducible protein (clone IFI -6-16) | MDK | Midkine (NEGF2) | Oxydative stress : | |
| IF127 | interferon, alpha-inducible protein 27 | TGFB2 | Transforming growth factor beta 2 | | |
| IF135 | interferon, alpha-inducible protein 35 | TNF | Tumor necrosis factor | CA9 | Carbonicanhydrase IX |
| IFIT1 | interferon induced protein with tetratricopeptide repeats 4 | TSLP | Thymic stromal lymphopoeitin | CYP2E1 | Cytochrome P450 CYP2E1 |
| IFIT4 | interferon induced protein with tetratricopeptide repents 1 | USP18 | ubiquitin-specific protease 18 | | |
| IFITM1 | interferon induced transmembrane protein 1 | | | Cell adhesion & cell junction | |
| LGALS3BP | lectin galactoside-binding soluble 3 | | | | |
| MX1 | myxovirus (influenza virus) resistance 1 | Angiogenesis | | ITGA2 | Integrin, alpha 2 |
| OAS1 | 2',5'-oligoadenylate synthetase 1 | | | | |
| OAS2 | 2'-5'-oligoadenylate synthetase 2 | FIGF | VEGF-D | Matrix proteases : | |
| OAS3 | 2',5',oligoadenylate synthetase 3 | | | | |
| PLSCR1 | phospholipid scramblase 1 | | | MMP9 | Matrix metalloproteinase 9 |
| PRKR | protein kinase IFN-inducible double stranded RNA dpt | Signal transduction | | ANPEP | alanyl aminopeptidase (CD13) |
| PSMB9 | proteasome (prosome,macroprain) subunit beta type 9 | | | | |
| HERC5 | Heat domain and RLD 5 | DUSP1 | Dual specificity phosphatase 1 (MKP-1) | Various : | |
| RSAD2 | radical s-adénosyl methionine domain containing 2 | EEFIG | eukaryotic translation elongation factor 1 g | | |
| | | | | CD14 | CD14 antigen |
| | | | | HLA-B | Major histocompatibility complex, class I, B |
| | | Cell cycle : | | LGP1 | D11 Igpte-like |
| Growth factors receptors : | | | | STXBP5 | syntaxin binding protein 5 (Tomosyn) |
| | | CDKN2A | CDK inhibitor 2A (p16 protein) | PIK3AP1 | phosphoinositide-3-kinase adaptator protein I |
| CXCR3 | Chemokine (C-X-C motif) receptor 3 | SFN | Stratifin (protein 14-3-3 sigma) | RPLP2 | ribosomal protein large P2 |
| | | | | RPS28 | ribosomal protein S28 |
| | | | | LAP3 | leucine aminopeptidase 3 |

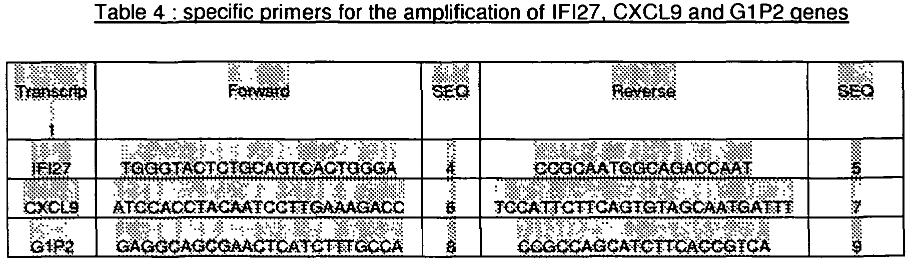

**Table 5. List of the genes that differ between NR and SVR hepatic gene expression profiles in Group A NOTE. The gene expression values of the samples were normalized such that the median of the normal histologic liver values was 1. Gene expression ratios were compared among NR and SVR liver gene expression values. Statistics are calculated using the Student's t-test. ^{a} Not adjusted for multiple testing**

| **Gene** | **NR** / **normal** | **SVR** / **normal** | **NR** / **SVR** | ***P*-value^{a}** | **FDR** |
|---|---|---|---|---|---|
| *G1P3* | 126.5 ± 84.3 | 35.6 ± 48.1 | 3.5 | 0.002 | 0.039 |
| *IFI27* | 141.1 ± 107.3 | 33.6 ± 40.7 | 4.2 | 0.002 | 0.039 |
| *G1P2* | 88.5 ± 80.3 | 24.1 ± 33.0 | 3.7 | 0.002 | 0.039 |
| *MIX1* | 40.1 ± 33.9 | 14.9 ± 19.2 | 2.7 | 0.006 | 0.059 |
| *HERC5* | 11.6 ± 9.1 | 5.4 ± 5.3 | 2.2 | 0.006 | 0.059 |
| *TGFB2* | 6.2 ± 7.6 | 2.3 ± 1.7 | 2.7 | 0.006 | 0.059 |
| *OAS2* | 27.0 ± 15.4 | 14.9 ± 16.0 | 1.8 | 0.016 | 0.118 |
| *VEGFD* | 5.5 ± 5.0 | 2.3 ± 2.9 | 2.4 | 0.020 | 0.118 |
| *IL8* | 49.2 ± 63.9 | 15.4 ± 15.5 | 3.2 | 0.020 | 0.118 |
| *IFIT1* | 387.8 ± 1399.3 | 7.0 ± 8.2 | 55.3 | 0.020 | 0.118 |

**Table 6. List of the genes that differ between NR and SVR hepatic gene expression profiles in Group B**

| NOTE. The gene expression values of the samples were normalized such that the median of the normal histologic liver values was 1. Gene expression ratios were compared among NR and SVR liver gene expression values. Statistics are calculated using the Student's *t*-test. ^{a} Not adjusted for multiple testing | | | | | |
|---|---|---|---|---|---|
| **Gene** | **NR** / **normal** | **SVR** / **normal** | **NR** / **SVR** | ***P*-value^{a}** | **FDR** |
| *IFI27* | 486.1 ± 370.7 | 95.8 ± 108.9 | 5.1 | 0.002 | 0.026 |
| *G1P2* | 341.3 ± 321.6 | 43.0 ± 39.2 | 7.9 | 0.002 | 0.026 |
| *G1P3* | 443.2 ± 569.6 | 78.3 ± 89.0 | 5.7 | 0.002 | 0.026 |
| *STAT1* | 33.3 ± 26.0 | 10.7 ± 9.3 | 3.1 | 0.002 | 0.026 |
| *CCL11* | 153.8 ± 123.7 | 49.2 ± 30.7 | 3.1 | 0.004 | 0.026 |
| *RPLP2* | 2.5 ± 2.3 | 0.7 ± 0.5 | 3.4 | 0.004 | 0.026 |
| *LGS3B* | 70.9 ± 70.6 | 15.0 ± 29.4 | 4.7 | 0.004 | 0.026 |
| *OAS2* | 79.5 ± 77.9 | 24.9 ± 26.5 | 3.2 | 0.004 | 0.026 |
| *HERC5* | 24.6 ± 28.2 | 5.5 ± 3.9 | 4.5 | 0.004 | 0.026 |
| *FGF7* | 34.3 ± 38.2 | 6.4 ± 5.9 | 5.3 | 0.006 | 0.035 |
| *ISF3G* | 19.7 ± 11.3 | 9.1 ± 7.2 | 2.2 | 0.008 | 0.039 |
| *RPS28* | 5.0 ± 5.9 | 1.5 ± 0.7 | 3.3 | 0.008 | 0.039 |
| *IFTM1* | 66.6 ± 71.1 | 15.6 ± 20.1 | 4.3 | 0.010 | 0.042 |
| *CXCL6* | 64.2 ± 86.9 | 17.2 ± 13.1 | 3.7 | 0.010 | 0.042 |
| *IFIT1* | 24.3 ± 20.8 | 9.1 ± 9.3 | 2.7 | 0.012 | 0.047 |
| *OAS3* | 227.1 ± 355.9 | 28.6 ± 34.4 | 7.9 | 0.014 | 0.049 |
| *IGP1* | 2.9 ± 2.8 | 1.1 ± 0.8 | 2.8 | 0.016 | 0.049 |
| *HLAB* | 72.5 ± 73.4 | 22.4 ± 33.7 | 3.2 | 0.016 | 0.049 |
| *PLCR1* | 36.1 ± 42.1 | 10.7 ± 11.3 | 3.4 | 0.016 | 0.049 |

**Table 7 : Models to predict treatment response**

| | Best selected model | NR predictive accuracy | SVR predictive accuracy | Overall predictive accuracy |
|---|---|---|---|---|
| *k-Nearest Neighbor* | | | | |
| Group A (training set, *n* = 40) | *IFI27, CXCL9* | 57.1% (8/14) | 88.5% (23/26) | 77.5% (31/40) |
| Group B (test set, *n* = 29) | | 100% (9/9) | 70.0% (14/20) | 79.3% (23/29) |
| Global (Group A + Group B, *n* = 69) | *IFI27, CXCL9^{a}* | 73.9% (17/23) | 84.8% (39/46) | 81.2% (56/69) |

| *Weighted Voting* | | | | |
|---|---|---|---|---|
| Group A (training set, *n* = 40) | *IFI27, CXCL9, G1P3* | 64.3% (9/14) | 88.5% (23/26) | 80.0% (32/40) |
| Group B (test set, *n* = 29) | | 88.9% (8/9) | 65.0% (13/20) | 72.4% (21/29) |
| Global (Group A + Group B, *n* = 69) | *IFI27, CXCL9, G1P2^{a}* | 60.9% (14/23) | 91.3% (42/46) | 81.2% (56/69) |

### REFERENCES:

Asselah T, Bieche I, Laurendeau I, et al. Liver gene expression signature of mild fibrosis in patients with chronic hepatitis C.Gastroenterology. 2005;129(6):2064-2075.
Bedossa P, Poynard T and The METAVIR Cooperative Study Group, An algorithm for the grading of activity in chronic hepatitis C, Hepatology 1996; 289-293.
Benjamini Y, Hochberg Y. Controlling the false discovery rate: a practical and powerfull approach to multiple testing. J R Statist Soc B 1995;57(1):289-300.
Bieche I, Asselah T, Laurendeau I, et al. Molecular profiling of early stage liver fibrosis in patients with chronic hepatitis C virus infection. Virology. 2005; 332(1):130-144.
Bigger C.B., Brasky K.M. and Lanford R.E. DNA microarray analysis of chimpanzee liver during acute resolving hepatitis C virus infection. J Virol 2001; 75, 7059-7066.
Chen L, Borozan I, Feld J, et al. Hepatic gene expression discriminates responders and nonresponders in treatment of chronic hepatitis C viral infection.Gastroenterology. 2005; 128(5):1437-1444.
Cover TM. Hart PE. Nearest neighbor pattern classification. IEEE Trans Info Theory 1967; IT-13:21-27.
Devlin B. Roeder K, Wasserman L. Analysis of multilocus models of association. Genet Epidemiol 2003;25:36-47.
EASL International Consensus Conference on Hepatitis C. Consensus Statement. J, Hepatol 1999; 30: 956-61.
Fried MW. Shiffman ML, Reddy KR, et al. Peginterferon alfa-2a plus ribavirin for chronic hepatitis C virus infection. N Engl J Med 2002; 347: 975-82.
Gale M Jr. Foy EM. Evasion of intracellular host defence by hepatitis C virus. Nature. 2005 Aug 18;436(7053):939-45.
Hadziyannis SJ, Sette H Jr, Morgan TR, et al. Peginterferon-alpha2a and ribavirin combination therapy in chronic hepatitis C: a randomized study of treatment duration and ribavirin dose. Ann Intern Med. 2004;140: 346-55.
Hayashida K, Daiba A, Sakai A, et al. Pretreatment prediction of interferon-alfa efficacy in chronic hepatitis C patients. Clin Gastroenterol Hepatol. 2005;3(12):1253-9.
Helbig KJ, Lau DT, Semendric L, Harley HA, Beard MR. Analysis of ISG expression in chronic hepatitis C identifies viperin as a potential antiviral effector. Hepatology. 2005; 42(3):702-10.
Honda M, Kaneko S, Kawai H, et al. Differential gene expression between chronic hepatitis B and C hepatic lesion, Gastroenterology 2001; 120, 955-966.
Kelly, J. M.; Porter, A. C. G.; Chernajovsky, Y.; Gilbert, C. S.; Stark, G. R.; Kerr, I. M. Characterization of a human gene inducible by alpha- and beta-interferons and its expression in mouse cells. EMBO J. 5: 1601-1606, 1986.
Lanford RE, Guerra B, Lee H, et al. Genomic response to interferon-alpha in chimpanzees: implications of rapid downregulation for hepatitis C kinetics. Hepatology. 2006;43(5):961-972.
Li Q, Zhang M, Kumar S, Zhu LJ, Chen D, Bagchi MK, Bagchi IC. Identification and implantation stage-specific expression of an interferon-alpha-regulated gene in human and rat endometrium. Endocrinology. 2001 Jun;142(6):2390-400.
Manns MP, McHutchison JG, Gordon SC, et al. Peginterferon alfa-2b plus ribavirin compared with IFN-2b plus ribavirin for initial treatment of chronic hepatitis C : a randomised trial. Lancet 2001; 358: 958-65.
Marcellin P, Asselah T and Boyer N. Fibrosis and disease progression in hepatitis C, Hepatology 2002; 36, S47-S56.
Martinot-Peignoux M, Boyer N, Pouteau M, et al. Predictors of sustaine response to alpha interferon therapy in chronic hepatitis C. J Hepatol 1998; 29: 214-23.
Mihm S, Frese M, Meier V, et al. Interferon type I gene expression in chronic hepatitis C. Lab Invest. 2004;84(9):1148-59.
National Institutes of Health consensus Development Conference Statement: management of Hepatitis C: 2002. Hepatology 2002; 36 (Suppl.1): S3-S21.
Neuman M.G., Benhamou J.P., Malkiewicz I.M, et al. Kinetics of serum cytokines reflect changes in the severity of chronic hepatitis C presenting minimal fibrosis, J Viral Hepat 2002; 9; 134-140.
Paradis V, Asselah T, Dargere D, et al. Surface-Enhanced Laser Desorption lonisation-Time-of-Flight (SELDI-TOF) ProteinChip to monitor and predict outcome in patients with chronic hepatitis C and combination therapy. Gastroenterology. 2006;130(7):2189-97.
Pockros PJ, Patel K, O'Brien C, et al. A multicenter study of recombinant human interleukin 12 for the treatment of chronic hepatitis C virus infection in patients nonresponsive to previous therapy, Hepatology 2003; 37; 1368-1374.
Rasmussen UB, Wolf C & Mattei M-G et al. Identification of a new interferon a-inducible gene (p27) on human chromosome 14q32 and its expression in breast carcinoma. Cancer Res (1993) 53: 4096-4101.
Reich M, Liefeld T, Gould J, Lerner J, Tamayo P, Mesirov JP. GenePattern 2.0 Nat Genet, 2006;38(5):500-501.
Seeff L B and Hoofnagle J H. The National Institutes of Health Consensus Development Conference Management of Hepatitis C 2002, Clin Liver Dis 2003; 7, 261-287.
Simmonds P, Bukh J, Combet C, Deléage G, Enomoto N, Feinstone S, Halfon P, Inchauspé G, Kuiken C, Maertens G, Mizokami M, Murphy D, Okamoto H, Pawlotsky J, Penin F, Sablon E, Shin-I T, Stuyver L, Thiel H, Viazov S, Weiner A, Widell A (2005). "Consensus proposals for a unified system of nomenclature of hepatitis C virus genotypes.". Hepatology 42 (4): 962-73.
Smith MW, Yue ZN, Korth MJ et al. Hepatitis C virus and liver disease global transcriptional profiling and identification of potential markers. Hepatology 2003; 38; 1458-1467.
Tahara E Jr, Tahara H, Kanno M, Naka K, Takeda Y, Matsuzaki T, Yamazaki R, Ishihara H, Yasui W, Barrett JC, Ide T, Tahara E. G1 P3, an interferon inducible gene 6-16, is expressed in gastric cancers and inhibits mitochondrial-mediated apoptosis in gastric cancer cell line TMK-1 cell. Cancer Immunol Immunother. 2005 Aug;54(8):729-40. Epub 2005 Feb 1.
Yeung E, Chung RT. Microarrays and prediction of interferon response in chronic hepatitis C: not yet ready for prime time. Clin Gastroenterol Hepatol. 2005;3(12):1192-4.
Zhao, C.; Denison, C.; Huibregtse, J. M.; Gygi, S.; Krug, R. M. Human G1P2 conjugation targets both IFN-induced and constitutively expressed proteins functioning in diverse cellular pathways. Proc. Nat. Acad. Sci. 102: 10200-10205, 2005.

## Claims

1. An in vitro method for determining whether a patient infected with HCV is a responder or a non-responder to a pharmaceutical treatment with a combination of interferon-alpha and ribavirin, the said method comprising: the steps of :
(a) determining the expression level of the IFI27 and CXCL9 marker genes in a biological sample previously collected from the said patient, and
(b) comparing, for each marker gene whose expression level has been determined at step (a), (i) the expression level determined at step (a) with (ii) a control reference value previously determined for the said gene in samples from HCV-infected patients selected from the group consisting of from responder patients, non-responder patients, or both.

2. The method according to claim 1, wherein step (a) further comprises determining the expression level of the G1 P2 marker gene.

3. The method of any one of claims 1 and 2, wherein the said patient is infected with an HCV of genotype 1.

4. The method of any one of claims 1 to 3, wherein, in the combination of interferon-alpha and ribavirin, the said interferon alpha is pegylated.

5. The method of claim 4, wherein, in the combination of interferon-alpha and ribavirin, the said interferon alpha is interferon alpha 2b

6. The method of any one of claims 1 to 5, wherein the said biological sample is selected from the group consisting of a liver biopsy, a blood sample or a serum sample.

7. The method of any of claims 1 to 6, wherein, at step (a), the expression level is determined by quantifying the level of mRNA of the said marker genes in the biological sample.

8. The method of claim 7, wherein step (a) comprises the steps of (a1) providing total RNAs collected from the said biological sample, and (a2) subjecting the RNAs to amplification and hybridization to probes specific for the said marker genes.

9. The method of any of claims 7 or 8, wherein, at step (a), the expression level is determined by real-time quantitative or semi-quantitative RT-PCR.

10. The method of any one of claims 1 to 6, wherein the expression level is determined, at step (a), by quantifying the level of the proteins encoded by the said marker genes in the said biological sample.

11. The method according to claim 10, wherein the quantification of the level of the proteins encoded by the said marker genes is performed by using a set of antibodies directed against each of the said proteins

## Patentansprüche

1. In-Vitro-Verfahren zur Bestimmung, ob ein HCV-infizierter Patient anspricht oder nicht anspricht auf eine pharmazeutische Behandlung mit einer Kombination von Interferon-Alpha und Ribavirin, wobei das Verfahren die folgenden Schritte aufweist:
(a) bestimmen des Expressions-Grades der IFI27 und CXCL9 Marker-Gene in einer biologischen Probe, die vorher vom genannten Patienten genommen wurde, und
(b) für jedes Marker-Gen, dessen Expressions-Grad im Schritt (a) bestimmt worden ist, vergleichen, (i) den Expressions-Grad, der im Schritt (a) bestimmt wurde, mit (ii) einem Kontroll-Referenzwert, der vorher bestimmt wurde für die genannten Gene in Proben von HCV-infizierten Patienten, die ausgewählt wurden aus einer Gruppe, die aus ansprechenden Patienten, nicht ansprechenden Patienten oder beiden besteht.

2. Verfahren nach Anspruch 1, wobei der Schritt (a) außerdem eine Bestimmung des Expressions-Grades des G1 P2 Marker-Gens umfasst.

3. Verfahren nach einem der Ansprüche 1 oder 2, wobei der genannte Patient infiziert ist mit einem HCV des Genomtyps 1.

4. Verfahren nach einem der Ansprüche 1 bis 3, wobei in der Kombination des Interferon-Alphas und Ribavirins das Interferon-Alpha pegyliert ist.

5. Verfahren nach Anspruch 4, wobei in der Kombination des Interferon-Alphas und Ribavirins das Interferon-Alpha ein Interferon-Alpha 2b ist.

6. Verfahren nach einem der Ansprüche 1 bis 5, wobei die genannte biologische Probe ausgewählt ist aus einer Gruppe, die besteht aus einer Leber-Biopsie, einer Blutprobe oder einer Serumprobe.

7. Verfahren nach einem der Ansprüche 1 bis 6, wobei beim Schritt (a) der Expressions-Grad bestimmt wird durch eine Quantifizierung des Levels der mRNA der genannten Marker-Gene in der biologischen Probe.

8. Verfahren nach Anspruch 7, wobei der Schritt (a) den Schritt (a1) aufweist, wonach die vollständigen RNAs vorgehalten werden von der vorgenannten biologischen Probe, und den Schritt (a2), in dem die RNAs einer Vergrößerung und einer Hybridisierung unterworfen werden zu Proben, die spezifisch sind für die genannten Marker-Gene.

9. Verfahren nach einem der Ansprüche 7 oder 8, wobei beim Schritt (a) der Expressions-Grad bestimmt wird durch eine Echtzeit-quantitative oder -semi-quantitative RT-PCR.

10. Verfahren nach einem der Ansprüche 1 bis 6, wobei der Expressions-Grad beim Schritt (a) bestimmt wird durch eine Quantifizierung des Levels der Proteine, die kodiert sind von den genannten Marker-Genen in der genannten biologischen Probe.

11. Verfahren nach Anspruch 10, wobei die Quantifizierung des Levels der Proteine, die kodiert sind durch die genannten Marker-Gene, ausgeführt wird durch die Benutzung einer Zusammenstellung von Antikörpern, die gegen jedes der genannten Proteine gerichtet sind.

## Revendications

1. Procédé in vitro pour déterminer si un patient infecté par le virus de l'hépatite C, (HCV), répond ou ne répond pas à un traitement pharmaceutique avec une combinaison de interféron-alpha et ribavirine, le procédé comprenant les étapes consistant à :
(a) déterminer le niveau d'expression des gènes marqueurs IF 127 et CXCL9 dans un échantillon biologique recueilli au préalable auprès du patient, et
(b) comparer, pour chaque gène marqueur dont le niveau d'expression a été déterminé à l'étape (a), (i) le niveau d'expression déterminé à l'étape (a) avec (ii) une valeur de référence témoin déterminée au préalable pour le gène dans des échantillons provenant de patients infectés par HCV sélectionnés parmi le groupe constitué de patients qui répondent, de patients qui ne répondent pas, ou des deux.

2. Procédé selon la revendication 1, dans lequel l'étape (a) comprend de plus la détermination du niveau d'expression du gène marqueur G1P2.

3. Procédé selon l'une quelconque des revendications 1 et 2, dans lequel le patient est infecté par un HCV de génotype 1.

4. Procédé selon l'une quelconque des revendications 1 à 3, dans lequel, dans la combinaison d'interféron-alpha et de ribavirine, l'interféron-alpha est pégylé.

5. Procédé selon la revendication 4, dans lequel, dans la combinaison d'interféron-alpha et de ribavirine, l'interféron-alpha est de l'interféron-alpha 2b.

6. Procédé selon l'une quelconque des revendications 1 à 5, dans lequel l'échantillon biologique est sélectionné parmi le groupe constitué d'une biopsie du foie, d'un échantillon de sang ou d'un échantillon de sérum.

7. Procédé selon l'une quelconque des revendications 1 à 6, dans lequel, à l'étape (a) le niveau d'expression est déterminé en quantifiant le niveau d'ARNm des gènes marqueurs dans l'échantillon biologique.

8. Procédé selon la revendication 7, dans lequel l'étape (a) comprend les étapes (a1) consistant à fournir les ARN totaux recueillis à partir de l'échantillon biologique, et (a2) soumettre les ARN à une amplification et une hybridation sur des sondes spécifiques pour les gènes marqueurs.

9. Procédé selon l'une quelconque des revendications 7 ou 8, dans lequel, à l'étape (a) le niveau d'expression est déterminé par une RT-PCR quantitative ou semi quantitative en temps réel.

10. Procédé selon l'une quelconque des revendications 1 à 6, dans lequel le niveau d'expression est déterminé, à l'étape (a), en quantifiant le niveau des protéines codées par les gènes marqueurs dans l'échantillon biologique.

11. Procédé selon la revendication 10, dans lequel la quantification du niveau des protéines codées par les gènes marqueurs est effectuée en utilisant un ensemble d'anticorps dirigé contre chacune des protéines.
